# EUROPEAN PATENT APPLICATION

(11) **EP 2 138 150 A1**
(43) Date of publication of application: **30.12.2009**
(21) Application number: 09151122.0
(22) Date of filing: 22.01.2009
(51) Int. Cl.: A61K 8/03, A61K 8/81, A61Q 5/00, A61Q 5/12

(54) **Personal care composition**

(30) Priority: 27.06.2008 EP 08159261
(71) Applicant: Unilever Plc, A Company Registered In England And Wales under company no. 41424 of Unilever House, London EC4Y 0DY Greater London (GB)
(72) Inventor: Ivanova, Katya, Merseyside CH63 3JW (GB); O' Neill, Niamh, Merseyside CH63 3JW (GB); Shaw, Neil Scott, Merseyside CH63 3JW (GB)
(74) Representative: Tansley, Sally Elizabeth

(57) **Abstract**

A multi-phase personal care composition comprising at least two visually distinct phases in physical contact with one another **characterised in that** at least one phase comprises polyacrylate-1 crosspolymer and a product appearance modifier and a separate phase comprises a conditioning lamellar phase.

## Description

The present invention relates to multi-phase personal care compositions, more particularly hair care compositions.

Conditioning formulations are, in general, non transparent and often packed into a non transparent packaging as well and, therefore, lacking the attractiveness, based on appearance judgment, to the consumer.

A problem with such conditioning products is that it is difficult to modify their appearance so that the product shimmers or pearlescers without using a large level of product appearance modifiers. Using large levels of these product appearance modifiers results in unacceptable cost and difficulties in product manufacture.

The present invention relates to conditioning compositions that have an attractive appearance without the need to use large levels of product appearance modifiers.

Accordingly the present invention relates to a multi-phase personal care composition comprising at least two visually distinct phases in physical contact with one another **characterised in that** at least one phase comprises polyacrylate-1 crosspolymer and a product appearance modifier and a separate phase comprises a conditioning lamellar phase.

Further described is a pack comprising a multi-phase composition as described above.

In the context of the present invention the term "visually distinct," means that the regions occupied by each phase can be separately seen by the human eye as distinctly separate regions in contact with one another (i.e. they are not emulsions or dispersions of particles of less than about 100 microns).

The term "multi-phased" or "multi-phase" as used herein, is meant that at least two phases occupy separate and distinct physical spaces inside the package in which they are stored, but are in direct contact with one another (i.e., they are not separated by a barrier).

In one preferred embodiment of the present invention, the "multi-phased" personal care compositions comprising at least two phases are present within the container as a visually distinct pattern. The pattern results from the mixing or homogenization of the "multi-phased" composition. The phases may be various different colours, or include particles, glitter or pearlescence.

In the context of present invention the term "visibly clear" means that the transmission of the composition is greater than 60%, preferably greater than 80%. The transparency of the composition is measured using Ultra-Violet/Visible (UV/VIS) Spectrophotometry, which determines the absorption or transmission of UV/VIS light by a sample. A light wavelength of 600 nm is known to be adequate for characterizing the degree of clarity of cosmetic compositions. Typically, it is best to follow the specific instructions relating the specific spectrophotometer being used. In general, the procedure for measuring percent transmittance starts by setting the spectrophotometer to the 600 nm. Then a calibration "blank" is run to calibrate the readout to 100 percent transmittance. The test sample is then placed in a cuvette designed to fit the specific spectrophotometer and the percent transmittance is measured by the spectrophotometer at 600 nm.

It is preferred if the multi-phase product has one phase that is visually clear. More preferred is a product having one visually clear phase and one opaque phase.

It is preferable if the differing phases of the composition are co-extruded.

Personal care compositions according to the invention comprise a polyacrylate-1 Crosspolymer. Poly-acrylate-1 Crosspolymer is a copolymer of one or more simple esters of acrylic or methacrylic acid, C1-4 dialkylamino C1-6 alkyl methacrylate, PEG/PPG-30/5 allyl ether, PEG 20-25 C10-30 alkyl ether methacrylate, hydroxyl C2-6 alkyl methacrylate crosslinked with ethylene glycol dimethacrylate. An especially preferred polyacrylate-1 Crosspolymer is Carbopol qua CC (ex Lubrizol/Noveon).

Compositions of the invention are usually aqueous. It is preferred if each phase comprising polyacrylate-1 crosspolymer further comprises water. The weight ratio of water to polyacrylate-1 crosspolymer is from 6:1 to 5:1.

The Compositions of the invention comprise a lamellar conditioning phase. Non-limiting examples of additives that promote thickening via lamellar structuring of surfactants for use in the personal care composition include fatty amides, fatty alcohols, fatty acid or ester derivatives thereof, electrolytes, and mixtures thereof. Examples of fatty acids which may be used are C10-C22 acids such as the following: lauric acid, oleic acid, isostearic acid, linoleic acid, linolenic acid, ricinoleic acid, elaidic acid, arichidonic acid, myristoleic acid, palmitoleic acid, and the like. Ester derivatives include propylene glycol, isostearate, propylene glycol oleate, glyceryl isostearate, glyceryl oleate, polyglyceryl diisostearate, and the like.

Suitable cationic surfactants for use in compositions of the invention, especially in the conditioning gel phase, contain amino or quaternary ammonium hydrophilic moieties which are positively charged when dissolved in the composition.

Suitable quaternary ammonium cationic surfactants correspond to the following general formula:

[N(R¹)(R²)(R³)(R⁴)]⁺ (X)⁻

in which R¹, R², R³, and R⁴ are each independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

In a suitable class of cationic surfactant of general formula (I), R¹ and R² are each independently selected from C₁₆ to C₂₂ hydrocarbyl chains comprising at least one ester linkage in both R¹ and R², and R³ and R⁴ are each independently selected from CH₃ and CH₂CH₂OH.

In another suitable class of cationic surfactant of general formula (I), R¹ and R² are each independently selected from C₁₆ to C₂₂ saturated or unsaturated, preferably saturated, chains, and R³ and R⁴ are each independently selected from CH₃ and CH₂CH₂OH, preferably CH₃.

In a preferred class of cationic surfactant of general formula (I), R¹ is a C₁₆ to C₂₂ alkyl chain and R², R³ and R⁴ are each independently selected from CH₃ and CH₂CH₂OH, preferably CH₃.

Specific examples of suitable quaternary ammonium cationic surfactants of general formula (I) are cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, dipalmitoylethyldimethylammonium chloride, PEG-2 oleylammonium chloride and salts of these, where the chloride is replaced by halogen, (e.g., bromide), acetate, citrate, lactate, glycolate, phosphate nitrate, sulphate, or alkylsulphate.

Particularly preferred quaternary ammonium cationic surfactants for use in the invention are cetyltrimethylammonium chloride, available commercially, for example as GENAMIN CTAC, ex Hoechst Celanese and Arquad 16/29 supplied by Akzo Nobel, and behenyltrimethylammonium chloride (BTAC) such as Genamin KDM-P supplied by Clariant.

Mixtures of any of the foregoing materials may also be suitable.

Salts of primary, secondary, and tertiary fatty amines are also suitable cationic surfactants for use in the invention. The alkyl groups of such amines preferably have from about 12 to about 22 carbon atoms, and can be substituted or unsubstituted. These amines are typically used in combination with an acid to provide the cationic species.

A preferred class of amine corresponds to the following general formula:

R¹ - C(O) - N(H)- R² - N(R³)(R⁴)

in which R¹ is a fatty acid chain containing from 12 to 22 carbon atoms, R² is an alkylene group containing from one to four carbon atoms, and R³ and R⁴ are, independently, an alkyl group having from one to four carbon atoms.

Specific examples of suitable materials of general formula (II) are stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, and diethylaminoethylstearamide.

Also useful are dimethylstearamine, dimethylsoyamine, soyamine, myristylamine, tridecylamine, ethylstearylamine, N-tallowpropane diamine, ethoxylated (with 5 moles of ethylene oxide) stearylamine, dihydroxyethylstearylamine, and arachidyl behenylamine.

Particularly preferred is stearamidopropyldimethylamine.

Mixtures of any of the foregoing materials may also be suitable.

The acid used to provide the cationic species can be any organic acid or mineral acid of sufficient acid strength to neutralise a free amine nitrogen. Such acids include hydrochloric acid, sulphuric acid, nitric acid, phosphoric acid, lactic acid, citric acid, tartaric acid, acetic acid, gluconic acid, glycolic acid and propionic acid, or combinations thereof. In general, a sufficient amount of acid is added to neutralise the amidoamine compound and to adjust the final pH of the composition to within a range of from about 2.5 to about 6, preferably in a pH range of from about 3 to about 5. The molar ratio of protonatable amine groups to H⁺ from the acid is preferably from about 1:0.3 to 1:1.2, and more preferably from about 1:0.5 to about 1:1.1.

Mixtures of any of the above-described cationic surfactants may also be suitable.

In the composition of the invention, the level of cationic surfactant preferably ranges from 0.1 to 10%, more preferably 0.2 to 5%, most preferably 0.25 to 4% by total weight of cationic surfactant based on the total weight of the composition.

A particularly preferred lamellar condition phase comprises cationic surfactant and fatty alcohol.

Preferably the composition is free of anionic surfactant.

Compositions of the invention preferably contain electrolytes. The electrolytes are preferably in the phase comprising the polyacrylate-1 cross polymer. Preferred electrolytes are metal and ammonium salts of carbonate and sulphate.
Particularly preferred is ammonium carbonate.

Preferably the level of electrolyte in the total composition is from 0.01 to 5 wt%, more preferably from 0.05 to 1 wt%.

Compositions of the invention may comprise an organic acid. Suitable organic acids include C1 to C5 organic acids. Preferred organic acids are hydroxyl acids such as lactic acid and glycolic acid. Glycolic acid is particularly preferred.

It is preferred if the weight ratio of polyacrylate crosspolymer to glycolic acid is from 4:1 to 1:1, more preferably from 3:1 to 1:1.

Compositions of the invention may comprise further conditioning agents to optimise wet and dry conditioning benefits.

Particularly preferred further conditioning agents are silicone emulsions.

Suitable silicone emulsions include those formed from silicones such as polydiorganosiloxanes, in particular polydimethylsiloxanes which have the CTFA designation dimethicone, polydimethyl siloxanes having hydroxyl end groups which have the CTFA designation dimethiconol, and amino-functional polydimethyl siloxanes which have the CTFA designation amodimethicone.

The emulsion droplets may typically have a Sauter mean droplet diameter (D_{3,2}) in the composition of the invention ranging from 0.01 to 20 micrometer, more preferably from 0.2 to 10 micrometer.

A suitable method for measuring the Sauter mean droplet diameter (D_{3,2}) is by laser light scattering using an instrument such as a Malvern Mastersizer.
Suitable silicone emulsions for use in compositions of the invention are available from suppliers of silicones such as Dow Corning and GE Silicones. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer. Pre-formed silicone emulsions having a Sauter mean droplet diameter (D_{3,2}) of less than 0.15 micrometers are generally termed microemulsions.

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions/microemulsions of dimethiconol. Also suitable are amodimethicone emulsions such as DC939 (from Dow Corning) and SME253 (from GE Silicones).

Also suitable are silicone emulsions in which certain types of surface active block copolymers of a high molecular weight have been blended with the silicone emulsion droplets, as described for example in WO03/094874. In such materials, the silicone emulsion droplets are preferably formed from polydiorganosiloxanes such as those described above. One preferred form of the surface active block copolymer is according to the following formula: 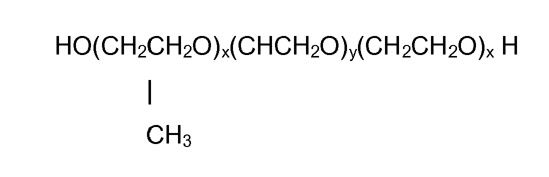 wherein the mean value of x is 4 or more and the mean value of y is 25 or more.

Another preferred form of the surface active block copolymer is according to the following formula: 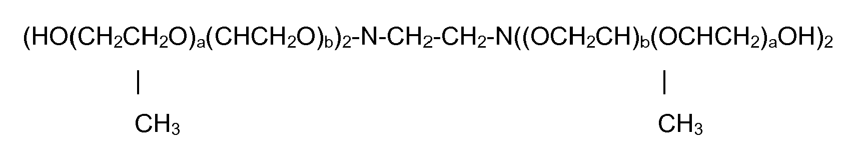 wherein the mean value of a is 2 or more and the mean value of b is 6 or more.

Mixtures of any of the above described silicone emulsions may also be used.

Silicone will generally be present in a composition of the invention at levels of from 0.05 to 10%, preferably 0.05 to 5%, more preferably from 0.5 to 2% by total weight of silicone based on the total weight of the composition.

If the product is a styling product it is preferred if a styling polymer is present.

The hair styling polymer if present is preferably present in the compositions of the invention in an amount of from 0.001 % to 10% by weight, more preferably from 0.1% to 10% by weight, such as from 1 % to 8% by weight.

Hair styling polymers are well known. Suitable hair styling polymers include commercially available polymers that contain moieties that render the polymers cationic, anionic, amphoteric or nonionic in nature. Suitable hair styling polymers include, for example, block and graft copolymers. The polymers may be synthetic or naturally derived.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

The final product form of hair treatment compositions according to the invention may suitably be, for example, conditioners, mousses, gels, waxes or lotions. Particularly preferred product forms are conditioners (leave-in and rinse-off) and hair leave in hair treatment products.

### Examples

The invention will now be further illustrated by the following, non-limiting Examples.

A number illustrates examples of the invention; a letter illustrates Comparative Examples.

All percentages quoted are by weight based on total weight unless otherwise stated.

### Example 1

### (one opaque and one shimmering phase)

| | **Raw Material** | **Supplier** | **Conc.** |
|---|---|---|---|
| | | | **w/w %** |
| **Phase 1** | | | |
| Aqua | Water | - | 41.705 |
| Lactic Acid | Purac HS 88 | Purac | 0.22 |
| Searamidopropyl Dimethylamine | Lexamine S13 | Inolex | 0.75 |
| Behentrimonium Chloride | Genamin BTLF | Clariant | 0.75 |
| Petrolatum | Petrolatum G 2212 | Sonneborn | 0.1 |
| Liquidum Parafifinum | White Mineral Oil | Sonneborn | 0.15 |
| Cetyl/Stearyl Alcohol | Lanette S3 | Cognis | 3 |
| PEG-180M | Polyox ™ WSR 308 | Amercol | 0.01 |
| Glycerin | Glycerin USP | Fluka | 0.5 |
| Potassium Chloride | Potassium Cloride, USP/Fcc | Sigma | 0.05 |
| Disodium EDTA | Disodium EDTA | Surfachem | 0.05 |
| Dimethyl Methyl (aminoethylaminoisobutyl) | DC 1785 | Dow Corning | 2.145 |
| Siloxane Parfum | Hyperion 85J | Givaudan | 0.5 |
| Methylchloroisothiazolinone and Methylisothiazolinone | Kathon CG | Rohm and Haas | 0.02 |
| DMDM Hydantoin | Glydant | Lonza | 0.05 |

| **Phase 2** | | | |
|---|---|---|---|
| Aqua | Water | - | 38.98 |
| Polyacrylate-1 Crosspolymer | Carbopol Aqua CC | Lubrizol | 7.5 |
| Glycolic Acid | Glycolic Acid | Sigma | 2.25 |
| Trehalose | Trehalose | Sigma | 0.6 |
| Ammonium Carbonate | Ammonium Carbonate | Sigma | 0.15 |
| Adipic Acid | Adipic Acid | Sigma | 0.15 |
| DMDM Hydantoin | Glydant | Lonza | 0.05 |
| Methylchloroisothiazolinone and Methylisothiazolinone | Kathon CG | Rohm and Haas | 0.02 |
| | Ronstar Golden Sparks | Merck | 0.3 |

## Claims

1. A multi-phase personal care composition comprising at least two visually distinct phases in physical contact with one another **characterised in that** at least one phase comprises polyacrylate-1 crosspolymer and a product appearance modifier and a separate phase comprises a conditioning lamellar phase.

2. A multi-phase personal care composition according to claim 1 in which the conditioning lamellar phase comprises a cationic conditioning ingredient.

3. A multi-phase personal care composition according to claim 1 or claim 2 in which at least one phase is visually clear.

4. A multi-phase personal care composition according to any preceding claim in which at least one phase is opaque.

5. A multi-phase care personal care composition according to any preceding claim in which at least one phase comprises electrolyte.

6. A multi-phase composition according to claim 5 in which the electrolyte and polyacrylate-1 crosspolymer are in the same phase.

7. A multi-phase personal care composition according to any preceding claim in which each phase comprising polyacrylate crosspolymer further comprises glycolic acid.

8. A multi-phase personal care composition according to claim 6 in which the ratio of polyacrylate crosspolymer to glycolic acid is from 3:1 to 1:1.

9. A multi-phase personal care composition according to any preceding claim in which each phase comprising polyacrylate-1 crosspolymer further comprises water and in which the weight ratio of water to polyacrylate-1 crosspolymer is from 6:1 to 5:1.

10. A multi-phase personal care composition according to any preceding claim for use on hair.

11. A personal care composition according to any preceding claim in which the differing phases of the composition are co-extruded.

12. A pack comprising a multi-phase composition as claimed in any one of claims 1 to 11.
